# EUROPEAN PATENT APPLICATION

(11) **EP 3 574 888 A1**
(43) Date of publication of application: **04.12.2019**
(21) Application number: 18020241.8
(22) Date of filing: 30.05.2018
(51) Int. Cl.: A61K 8/41, A61K 8/34, A61K 8/35, A61K 8/40, A61K 8/368, A61K 8/37, A61K 8/36, A61K 8/44, A61K 8/43, A61Q 19/00

(54) **COSMETIC PRESERVATIVE SYSTEM**

(71) Applicant: The Boots Company PLC, Nottingham Nottinghamshire NG2 3AA (GB)
(72) Inventor: Tomlinson, Paul James, Darley Abbey, DERBY DE22 1EX (GB); Johnson, Mark, Hucknall, NOTTINGHAM NG15 8DQ (GB); Hicks, Jake Thomas, Chilwell, NOTTINGHAM NG9 6RR (GB)
(74) Representative: Goodier, Claire-Louise

(57) **Abstract**

According to the present invention there is provided a cosmetic composition comprising a cosmetically acceptable carrier and a preservative system, wherein the preservative system comprises:
(i) an antimicrobial agent;
(ii) a chelating agent comprising ethylenediamine tetraacetic acid (EDTA) or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% by weight of the composition.

## Description

### Field of the invention

The present invention relates to novel preservative systems and the use of these systems in cosmetics.

### Background of the invention

Microbial contamination of cosmetic products is problematic and is a major cause of product recall and industry economic loss. Cosmetic products are ideal substrates for the survival and development of a large variety of microorganisms, since they possess water and many nutrients that facilitate their growth¹.

The presence of certain microorganisms in cosmetic products can pose a health risk for consumers, for example in the form of skin irritation, allergic contact dermatitis and infection, especially in the eyes, mouth or wounds. Moreover, the presence of microorganisms can negatively affect the organoleptic properties, e.g. the smell, viscosity and colour, of cosmetic products.

The use of preservatives that are able to reduce microbial load to acceptable levels has improved the microbiological quality of cosmetics. Regulations in the EU and in other countries provide lists of allowed preservatives with maximum cosmetic use concentrations. However, preservatives are known as one of the most relevant allergens found in cosmetic products, themselves causing contact dermatitis in some susceptible users. Further, despite the diversity of preservatives that can be used, the contamination frequency of cosmetics by certain bacteria is recurrent.

A particular problematic and recurring contaminant is the Gram-negative bacteria *Enterobacter gergoviae,* (also known as *Pluralibacter gergoviae*) which is associated with a number of human infectious diseases.

*E. gergoviae* is often identified by quality control laboratories in recently manufactured or spoiled cosmetic products. *E. gergoviae* is ubiquitous in the environment and the origin of contaminations of cosmetics by this species varies largely, meaning it has the potential to contaminate cosmetic products during manufacture, storage and use. The majority of *E. gergoviae* strains isolated from cosmetics are also unrelated.

It has been shown that *E. gergoviae* shows significant levels of resistance to a variety of preservatives. For example, *E. gergoviae* exhibits an innate resistance to parabens, due to the production of an enzyme PrbA and an efflux mechanism². Efflux mechanisms are also likely to be involved in methylisothiazolinone, methylchloroisothiazolinone and triclosan adaptation to *E. gergoviae².* Further, the maximum allowed preservative concentrations for sodium benzoate are inefficient to limit proliferation and control adaptability to *E. gergoviae* in cosmetic products².

Preservative misuse/overuse is a key factor leading to the selection of preservative-resistant bacteria, such as *E. gergoviae.* Without an effective preservative system, cosmetic products susceptible to *E. gergoviae* growth and proliferation are likely to soon have little or no protection against it, leaving products unsafe for human use.

### Summary of invention

The Applicants have identified a consumer need to provide cosmetic preservative systems with improved antimicrobial properties, particularly against *E. gergoviae,* whilst being mild for skin tolerance. The Applicants have found that the preservative systems used in accordance with the present invention provide good and effective benefits in protecting against *E. gergoviae* as well as standard challenge test microorganisms (e.g. *Pseudomonas aeruginosa, Staphylococcus aureus, Escherichia coli, Candida albicans,* and *Aspergillus brasiliensis*) in cosmetic products.

Accordingly, in a first aspect of the invention there is provided a cosmetic composition comprising:
(i) an antimicrobial agent;
(ii) a chelating agent comprising ethylenediamine tetraacetic acid (EDTA) or a derivative thereof;
(iii) an antimicrobial booster agent selected from the group consisting of ethylhexylglycerin, caprylyl glycol, hydroxy acetophenone, caprylhydroxamic acid, phenethyl alcohol, propanediol, p-anisic acid, glyceryl caprylate, levulinic acid, sodium levulinate, hexanediol, pentylene glycol, capryloyl glycine, methylpropanediol, phenylpropanol, sodium anisate, and combinations thereof; and
(iv) a cosmetically acceptable carrier,
wherein the antimicrobial agent is present in an amount of from about 0.001% to about 1% by weight of the composition.

In another aspect of the invention there is provided a cosmetic composition comprising a cosmetically acceptable carrier and a preservative system, wherein the preservative system comprises:
(i) an antimicrobial agent;
(ii) a chelating agent comprising ethylenediamine tetraacetic acid (EDTA) or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% by weight of the composition.

In another aspect of the invention there is provided a use of a preservative system to prevent or reduce *Enterobacter gergoviae* growth or proliferation in a cosmetic composition, wherein the preservative system comprises:
(i) an antimicrobial agent;
(ii) a chelating agent comprising ethylenediamine tetraacetic acid (EDTA) or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% by weight of the composition.

The present invention is characterised by a preservative system/preservative ingredients having an antimicrobial effect against the problematic and highly preservative-resistant *E.gergoviae,* being capable of maintaining the bacterial load in cosmetic products to below the level of acceptability according to microbiological international standards for cosmetics. When used in cosmetic products, the preservative system additionally leads to products which are well tolerated by skin and keeps unchanged the organoleptic properties of the cosmetic products.

Surprisingly, it has been found that use of a specific combination of an antimicrobial agent, a chelating agent and an antimicrobial booster agent in accordance with the present invention results in a good antimicrobial effect against *E.gergoviae.* The combination allows formulation of an effective preservative system with lower overall levels of antimicrobials in the end cosmetic product. Despite the overall antimicrobial level being low, adequate protection against *E.gergoviae* and standard challenge test microorganisms can be achieved during manufacture, storage and use. Advantageously, the reduced level of antimicrobial agent used is well tolerated on the skin and may reduce or prevent the allergic or irritating effects often associated with high levels of preservatives in cosmetic products. Further, the problems of bacterial adaptation and resistance to preservative-resistant bacteria such as *E.gergoviae* will also likely be reduced.

### Detailed description of the invention

The invention makes use of an antimicrobial agent. The term "antimicrobial agent" is intended to mean an agent which provides an antimicrobial benefit as would be understood by the skilled person. For example, an agent that is capable of preventing or reducing the growth or proliferation of and/or killing microorganisms such as bacteria and fungi.

The antimicrobial agent may comprise any one or more of the recognised preservatives allowed in cosmetic products as would be understood by the skilled person. For example, the antimicrobial agent may be selected from the group consisting of chlorhexidine, chlorhexidine digluconate, chlorhexidine dihydrochloride, chlorhexidine diacetate, chlorhexidine gluconate, chlorhexidine hydrochloride, chlorhexidine phosphanilate, chlorphensin, benzoic acid or a salt or ester thereof (e.g. sodium benzoate), propionic acid or a salt thereof, salicylic acid or a salt thereof, sorbic acid or a salt thereof (e.g. potassium sorbate), formaldehyde, paraformaldehyde, zinc pyrithione, inorganic sulphites, hydrogen sulphites, chlorobutanol, 4-hydroxybenzoic acid or a salt or ester thereof (e.g. methylparaben, ethylparaben, propylparaben), dehydroacetic acid and/or a salt thereof (e.g. sodium dehydroacetate), formic acid or a salt thereof, dibromohexamidine isethionate; thimerosal, phenylmecuric salts, undecylenic acid or a salt thereof, hexetidine, bronopol, 5-bromo-5-nitro-1,3-dioxane, dichlorobenzyl alcohol, benzyl alcohol, triclocarban, chlororesol, triclosan, chloroxylenol, imidazolidinyl urea, polyaminopropyl biguanide, phenoxyethanol, methenamine, quaternium-15, climbazole, DMDM hydantoin, 1-hydroxy-4-methyl-6-(2,4,4-trimethylenepentyl)-2 pyridon, piroctone olamine, bromochlorophene, O-cymen-5-ol, methylchloroisothiazolinone, methylisothiazololinone, mixtures of methylchloroisothiazolinone and methylisothiazololinone, chlorophene, chloroacetamide, phenoxyisoproponol, alkyl (C12-C22) trimethyl ammonium bromide, alkyl (C12-C22) trimethyl ammonium chloride, dimethyl oxazolidine, diazolidinyl urea, hexamidine, hexamidine diisethionate, hexamidine diparaben, hexamidine paraben, glutaral, 7-ethylbicyclooxazolidine, sodium hydroxymethylaminoacetate, silver chloride, benzethonium chloride, benzalkonium chloride, benzalkonium bromide, benzalkonium saccharinate, benzylhemiformal, iodopropynyl butylcarbamate, silver citrate, and combinations thereof.

In one embodiment, the antimicrobial agent is selected from the group consisting of chlorhexidine, chlorhexidine digluconate, chlorphensin, benzoic acid or a salt or ester thereof (e.g. sodium benzoate), sorbic acid or a salt thereof (e.g. potassium sorbate), 4-hydroxybenzoic acid or a salt or ester thereof (e.g. methylparaben, ethylparaben, propylparaben), dehydroacetic acid or a salt thereof (e.g. sodium dehydroacetate), bronopol, benzyl alcohol, polyaminopropyl biguanide, phenoxyethanol, and combinations thereof.

In one embodiment, the antimicrobial agent is selected from the group consisting of chlorhexidine digluconate, chlorphensin, benzoic acid, sodium benzoate, potassium sorbate, dehydroacetic acid and/or a salt thereof (e.g. sodium dehydroacetate), polyaminopropyl biguanide, phenoxyethanol, or combinations thereof.

In one embodiment, the antimicrobial agent does not comprise any parabens e.g. methylparaben, ethylparaben, and/or propylparaben.

In one embodiment, the antimicrobial agent comprises potassium sorbate, polyaminopropyl biguanide, chlorphensin, sodium benzoate, chlorhexidine digluconate, or combinations thereof.

In one embodiment, the antimicrobial agent comprises or consists of (i) one or more of phenoxyethanol, dehydroacetic acid, sodium dehydroacetate, or benzoic acid and (ii) one or more of potassium sorbate, polyaminopropyl biguanide, chlorphensin, sodium benzoate, or chlorhexidine digluconate.

The antimicrobial agent may be present in an amount from about 0.01% to about 1% by weight relative to the total weight of the composition, for example from about 0.05% to about 1% by weight of the composition, or from about 0.1% to about 1% by weight of the composition, or from about 0.25% to about 1% by weight of the composition, or from about 0.3% to about 0.95% by weight of the composition, or from about 0.5% to about 0.9% by weight of the composition, or from about 0.6 to about 0.85% by weight of the composition.

The total amount of antimicrobial agent present in the cosmetic composition of the invention may be about 1% or less by weight relative to the total weight of the composition, for example present at a level of about 0.95% or less, or about 0.9% or less, or about 0.85% or less, or about 0.8% or less relative to the total weight of the composition.

In one embodiment, the cosmetic composition of the invention does not further comprise any additional antimicrobial agents. In one embodiment, the only antimicrobial agents present in the cosmetic composition of the invention are those present in the preservative system.

The invention makes use of a chelating agent. The term "chelating agent" is intended to mean an agent which is capable of forming complexes with metal ions as would be understood by the skilled person.

The chelating agent may be selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof. In one embodiment, the chelating agent comprises tetrasodium EDTA.

The chelating agent may be present in an amount from about 0.001% to about 2% by weight relative to the total weight of the composition, for example from about 0.001% to about 1.5%, or from about 0.005% to about 1%, or from about 0.008% to about 0.8%, or from about 0.01% to about 0.5%, or from about 0.01% to about 0.25%. In one embodiment, the chelating agent is present in amount from about 0.02% to about 0.2%.

In one embodiment, the cosmetic composition of the invention does not further comprise any additional chelating agents. In one embodiment, the only chelating agent present in the cosmetic composition of the invention is that which is present in the preservative system.

In the preservative system, the antimicrobial agent may be present in an amount of at least the same amount as the chelating agent. In one embodiment, the antimicrobial agent is present in an amount of about 20 times or less the amount of the chelating agent, for example about 19.5 times or less, about 19 times or less, about 18.5 times or less, about 18 times or less, about 17.5 times or less, about 17 times or less, about 16.5 times or less, about 16 times or less, or about 15.5 times or less.

The invention makes use of an antimicrobial booster agent. Advantageously, use of an antimicrobial booster agent in the present invention allows for better formulation of the composition and may further improve the activity of the antimicrobial agent.

In a preferred embodiment the antimicrobial booster agent comprises ethylhexylglycerin, caprylyl glycol, hydroxy acetophenone, caprylhydroxamic acid, phenethyl alcohol, propanediol, or combinations thereof.

The antimicrobial booster agent may be present in an amount from about 0.001% to about 2% by weight of the cosmetic composition of the invention, or about 0.001% to about 1% by weight of the composition, or about 0.005% to about 1% by weight of the composition, or about 0.01% to about 0.8% by weight of the composition. In one embodiment, the antimicrobial booster agent comprises ethylhexylglycerin, optionally in an amount of from about 0.01% to about 0.5% by weight of the composition.

The combination of the antimicrobial agent, chelating agent and antimicrobial booster agent in accordance with the present invention is surprisingly effective in reducing or preventing growth or proliferation of *E.gergoviae* in a cosmetic product. The combination is also effective against the standard challenge test microorganisms *Pseudomonas aeruginosa, Staphylococcus aureus, Escherichia coli, Candida albicans,* and *Aspergillus brasiliensis.* What is particularly unexpected is that despite the individual and/or overall levels of antimicrobials being reduced compared to the levels used conventionally in cosmetic products, the cosmetic composition of the invention is still just as effectively if not better preserved and protected from contaminants. As a result, the cosmetic composition will be better tolerated on the skin and may reduce or prevent the allergic or irritating effects often associated with higher levels of preservatives in cosmetic products. Therefore there is a technical benefit to using the combination of the antimicrobial agent, chelating agent and antimicrobial booster agent in a single formulation that would not have been foreseen.

The cosmetic composition of the invention may comprise the preservative system in an amount effective to achieve an 'A criteria pass' for bacteria, yeast and mould (e.g. *E.gergoviae, Pseudomonas aeruginosa, Staphylococcus aureus, Escherichia coli, Candida albicans,* and *Aspergillus brasiliensis*) in accordance with the pass acceptance criteria outlined in the EU Pharmacopeia, section 5.1.3, and also discussed in the Examples section below.

For example, for bacteria (e.g. *E.gergoviae*)*,* the cosmetic composition may comprise the preservative system in an amount effective to produce at least a 2 log₁₀ reduction in the number of viable/live bacteria against the value obtained for the inoculum at a time point of 2 days after inoculation of the cosmetic composition and/or an amount effective to produce at least a 3 log₁₀ reduction in the number of viable/live bacteria against the value obtained for the inoculum at a time point of 7 days after inoculation of the cosmetic composition.

Additionally or alternatively, the cosmetic composition may comprise the preservative system in an amount effective to produce less than 100 viable microorganisms (e.g. *E.gergoviae, Pseudomonas aeruginosa, Staphylococcus aureus, Escherichia coli, Candida albicans,* and *Aspergillus brasiliensis*) on a plate count two days after inoculation of the composition with about 10⁵ to about 10⁶ per mL of said microorganism. Additionally or alternatively, the cosmetic composition may comprise the preservative system in an amount effective to produce less than 10 viable microorganisms on a plate count seven days after inoculation of the composition with about 10⁵ to about 10⁶ per mL of said microorganism. Additionally or alternatively, the cosmetic composition may comprise the preservative system in an amount effective to produce less than 10 viable microorganisms on a plate count fourteen days after inoculation of the composition with about 10⁵ to about 10⁶ per mL of said microorganism. Additionally or alternatively, the cosmetic composition may comprise the preservative system in an amount effective to produce less than 10 viable microorganisms on a plate count twenty eight days after inoculation of the composition with about 10⁵ to about 10⁶ per mL of said microorganism.

The present invention provides the following embodiments.

In one embodiment, the cosmetic composition of the invention comprises a cosmetically acceptable carrier and a preservative system, wherein the preservative system comprises:
(i) an antimicrobial agent;
(ii) a chelating agent selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% (e.g. from about 0.01% to about 1%, or from about 0.1% to about 1%) by weight of the composition.

In one embodiment, the cosmetic composition of the invention comprises a cosmetically acceptable carrier and a preservative system, wherein the preservative system comprises:
(i) an antimicrobial agent;
(ii) a chelating agent comprising at least one of disodium EDTA, trisodium EDTA, tetrasodium EDTA, or combinations thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% (e.g. from about 0.01% to about 1%, or from about 0.1% to about 1%) by weight of the composition.

In one embodiment, the cosmetic composition of the invention comprises a cosmetically acceptable carrier and a preservative system, wherein the preservative system comprises:
(i) an antimicrobial agent comprising potassium sorbate, chlorphensin, sodium benzoate, chlorhexidine digluconate, phenoxyethanol, benzoic acid, dehydroacetic acid, sodium dehydroacetate, polyaminopropyl biguanide, or combinations thereof;
(ii) a chelating agent comprising EDTA or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% (e.g. from about 0.01% to about 1%, or from about 0.1% to about 1%) by weight of the composition.

In one embodiment, the cosmetic composition of the invention comprises a cosmetically acceptable carrier and a preservative system, wherein the preservative system comprises:
(i) an antimicrobial agent comprising or consisting of (a) one or more of phenoxyethanol, dehydroacetic acid or a salt thereof, sodium dehydroacetate, or benzoic acid and (b) one or more of potassium sorbate, polyaminopropyl biguanide, chlorphensin, sodium benzoate, or chlorhexidine digluconate;
(ii) a chelating agent comprising EDTA or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% (e.g. from about 0.01% to about 1%, or from about 0.1% to about 1%) by weight of the composition.

In one embodiment, the cosmetic composition of the invention comprises a cosmetically acceptable carrier and a preservative system, wherein the preservative system comprises:
(i) an antimicrobial agent selected from the group consisting of chlorhexidine or a salt thereof, chlorphensin, benzoic acid or a salt or ester thereof (sodium benzoate), sorbic acid or a salt thereof (e.g. potassium sorbate), 4-hydroxybenzoic acid or a salt or ester thereof (e.g. methylparaben, propylparaben), dehydroacetic acid and/or a salt thereof (e.g. sodium dehydroacetate), bronopol, polyaminopropyl biguanide, phenoxyethanol, and combinations thereof;
(ii) a chelating agent selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof
wherein the antimicrobial agent is present in an amount of from about 0.001% to about 1% (e.g. from about 0.01% to about 1%, or from about 0.1% to about 1%) by weight of the composition and/or the chelating agent may be present in an amount of from about 0.001% to about 2% (e.g. from about 0.007 to about 0.7%, or from about 0.02% to about 0.2%) by weight of the composition. In one such embodiment, the only antimicrobial agent present in the cosmetic composition may be that which is present in the preservative system.

In one embodiment, the cosmetic composition of the invention comprises a cosmetically acceptable carrier and a preservative system, wherein the preservative system comprises:
(i) an antimicrobial agent selected from the group consisting of chlorhexidine or a salt thereof, chlorphensin, benzoic acid or a salt or ester thereof (sodium benzoate), sorbic acid or a salt thereof (e.g. potassium sorbate), 4-hydroxybenzoic acid or a salt or ester thereof (e.g. methylparaben, propylparaben), dehydroacetic acid and/or a salt thereof (e.g. sodium dehydroacetate), bronopol, polyaminopropyl biguanide, phenoxyethanol, and combinations thereof;
(ii) a chelating agent comprising tetrasodium EDTA; and
(iii) an antimicrobial booster agent selected from the group consisting of ethylhexylglycerin, caprylyl glycol, hydroxy aceetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% (e.g. from about 0.01% to about 1.3%, or from about 0.1% to about 1%) by weight of the composition,
optionally wherein the chelating agent is present in an amount from about 0.001% to about 2% (e.g. from about 0.007 to about 0.7%, or from about 0.02% to about 0.2%) by weight of the composition,
optionally wherein the antimicrobial booster agent is present in an amount of from about 0.001% to about 2% (e.g. from about 0.005% to about 0.5%, or from about 0.01 to about 0.5%) by weight of the composition, and
optionally wherein the only antimicrobial agent present in the cosmetic composition of the invention is that which is present in the preservative system.

A cosmetic composition comprising:
(i) an antimicrobial agent;
(ii) a chelating agent selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof;
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof; and
(iv) a cosmetically acceptable carrier,
wherein the antimicrobial agent is present in an amount of from about 0.001% to about 1% by weight of the composition,
optionally wherein the chelating agent is present in an amount from about 0.001% to about 2% (e.g. from about 0.007 to about 0.7%, or from about 0.02% to about 0.2%) by weight of the composition, and
optionally wherein the antimicrobial booster agent is present in an amount of from about 0.001% to about 2% (e.g. from about 0.005% to about 0.5%, or from about 0.01 to about 0.5%) by weight of the composition.

A cosmetic composition comprising:
(i) an antimicrobial agent selected from the group consisting of chlorhexidine or a salt thereof, chlorphensin, benzoic acid or a salt or ester thereof (e.g. sodium benzoate), sorbic acid or a salt thereof (e.g. potassium sorbate), 4-hydroxybenzoic acid or a salt or ester thereof (e.g. methylparaben, propylparaben), dehydroacetic acid and/or a salt thereof (e.g. sodium dehydroacetate), bronopol, polyaminopropyl biguanide, phenoxyethanol, and combinations thereof;
(ii) a chelating agent selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof;
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof; and
(iv) a cosmetically acceptable carrier,
wherein the antimicrobial agent is present in an amount of from about 0.001% to about 1% by weight of the composition,
optionally wherein the chelating agent is present in an amount from about 0.001% to about 2% (e.g. from about 0.007 to about 0.7%, or from about 0.02% to about 0.2%) by weight of the composition,
optionally wherein the antimicrobial booster agent is present in an amount of from about 0.001% to about 2% (e.g. from about 0.005% to about 0.5%, or from about 0.01 to about 0.5%) by weight of the composition, and
optionally wherein the cosmetic composition does not further comprise any additional antimicrobial agents.

### Cosmetic composition

The invention makes use of a cosmetically acceptable carrier. The carrier may be water-based, oil-based, or emulsion-based.

In embodiments where the carrier is emulsion-based, the composition may be in the form of a water-in-oil, an oil-in-water, a water-in-oil-in-water or a oil-in-water-in-oil emulsion.

In embodiments where the carrier is water-based, water may be present at a level of about 40% or more, about 45% or more, about 50% or more, about 55% or more, or about 60% or more by weight of the composition.

The cosmetic composition of the invention may be provided in any form suitable for topical application to the skin and/or hair. The cosmetic composition of the invention may be delivered and/or applied to the skin and/or hair via any of the conventional formulations known to those skilled in the art. Typical formulation types of the present invention are liquids, creams, lotions, milks, mousses, gels, sprays, serum, foams, aerosols, and ointments.

In embodiments where the cosmetic composition is in the form of a water-in-oil emulsion, water may be present at a level of about 20% to about 60% by weight of the composition, about 20% to about 50% by weight of the composition, or about 35% to about 45% by weight of the composition. In one embodiment where the cosmetic composition is in the form of a water-in-oil emulsion, water is present at a level of about 35% to about 45% by weight of the composition.

In embodiments where the cosmetic composition is in the form of an oil-in-water emulsion, water may be present in an amount of about 40% to about 90% by weight of the composition or about 60% to about 95% by weight of the composition. In one embodiment where the cosmetic composition is in the form of an oil-in-water emulsion, water is present at a level of about 60% to about 95% by weight of the composition.

In addition to the carrier and the preservative system, the cosmetic composition of the invention will generally further comprise other ingredients or excipients which will be well known to those skilled in the art.

The cosmetic composition of the invention may further comprise one or more humectants, including but not limited to glycerin, propylene glycol, butylene glycol, hexylene glycol, dipropylene glycol, polyethylene glycol, sorbitol, sodium hyaluronate, urea, xylitol, lactate, fructose, glucose, mannose, xylose, honey, pyrrolidone, and carboxylic acid and salts thereof. When present, the one or more humectants may be present in the cosmetic composition in an amount of about 0.01% to about 20% by weight of the composition, about 0.1% to about 10%, or about 0.5% to about 7% by weight of the composition.

The cosmetic composition of the invention may further comprise one or more emollients, including but not limited to cetyl esters, cera alba, PPG-15 stearyl ether, ethylhexyl stearate, cetyl dimethicone, octyldodecanol, PPG-20 methyl glucose ether, isopropyl myristate, isopropyl paltimate, isopropyl laurate, isodecyl laurate, isodecyl neopentanoate, isohexadecane, pentaerythrityl tetraisostearate, caprylic/capric triglyceride, canola oil, sunflower oil (*Helianthus annus*), olive oil (*Olea europea*), cottonseed oil (*Gossypium herbaceum*), jojoba oil (*Simmondsia chinensis*)*,* shea butter (*Butyrospermum parkii*)*,* cocoa butter (*Theobroma cacao*)*,* cupuacu butter (*Theobroma grandiflorum*)*,* avocado oil (*Persea gratissima*)*,* liquid paraffin, dimethicone, phenyl trimethicone, cyclopentasiloxane, dimethiconol and petrolatum. When present, the one or more emollients may be present in the cosmetic composition in an amount of about 0.01% to about 20% by weight of the composition, about 0.1% to about 10%, or about 0.5% to about 7% by weight of the composition.

The cosmetic composition may further comprise one or more emulsifiers, including but not limited to steareth-2, steareth-21, steareth-10, ceteareth-5, ceteareth-20, cetearyl glucoside, oleth-10, glyceryl stearate, polyglycerol-3 oleate, polyglyceryl-3 methylglucose distearate, , sodium stearate, PEG-60, PEG-12 oleate, PEG-2 stearate, PEG-12 stearate, PEG-20 stearate, PEG-100 stearate, polysorbate-60, cetyl alcohol, cetearyl alcohol, potassium cetyl phosphate, cetearyl olivate, sorbitan olivate, PEG-80 sorbitan, sorbitan oleate, sorbitan stearate, and/or sorbitan palmitate. In one embodiment, the cosmetic composition of the invention does not comprise sulphates as emulsifiers. In embodiments where one or more emulsifiers are present in the cosmetic composition, the one or more emulsifiers may be present in an amount of about 0.01% to about 5% or about 0.01% to about 2% by weight of the composition. In one embodiment, the cosmetic composition of the invention does not contain emulsifiers.

The cosmetic composition of the invention may further comprise one or more surfactants, including but not limited to, anionic surfactants (e.g. sodium lauryl sulphate, sodium laureth sulphate, ammonium laureth sulphate, disodium laureth sulfosuccinate and sodium C12-15 pareth-12 carboxylate), amphoteric/zwitterionic surfactants (e.g. cocamidopropyl betaine, sodium cocoamphoacetate and cocamidopropyl hydroxysultaine), non-ionic surfactants (e.g. cocamide DEA, cocamide MEA, decyl glucoside, lauryl glucoside), and cationic surfactants (e.g. cetrimonium chloride, behentrimonium chloride and benzalkonium chloride). In embodiments where one or more surfactants are present in the cosmetic composition, the one or more surfactants may be present in an amount of from about 0.1% to about 10% by weight of the composition, e.g. from about 0.25% to about 7.5% by weight of the composition, or about 0.5% to about 6% by weight of the composition. In one embodiment where one or more surfactants are present in the cosmetic composition, the one or more surfactants are present in an amount of from about 0.5% to about 5% by weight of the composition.

The cosmetic composition of the invention may further comprise one or more antioxidant agents, for example a polyphenolic antioxidant agent selected from the group consisting of extracts of mulberry (e.g. *Morus alba*)*,* ginseng (e.g. *Panax ginseng*)*,* raspberry, oregano (e.g. *Origanum vulgare*)*,* green tea (e.g. green leaves of *Camellia sinensis*)*,* white tea (e.g. *Camellia sinensis*)*,* blueberry (e.g. *Vaccinium cyanococcus*), *Eucalyptus globulus,* chamomile (e.g. *Anthenis nobilis*), French maritime pine bark (e.g. *Pinus pinaster,* sold under the tradename of Pycnogenol), rosemary (e.g. *Rosemarinus officialis*), grape, including grape seed (e.g. *Vitis vinifera),* fennel (e.g. *Foeniculi fructus), Caragana sinica,* majaoram (e.g. *Origanum majorana*), crocus (e.g. *Crocus sativus*), apple (e.g. *Malus domestica*), coffee, green coffee, cherry (e.g. *Prunus avium*), snow algae (e.g. *Chlamydomonas nivalis*), Emblica (e.g. *Pyllanthus emblica*), ginkgo (e.g. *Ginkgo biloba*), moringa (e.g. *Moringa oleilera*), ginger, magnolia (e.g. *Magnolioideae virginiana*), French saffron, edelweiss (e.g. *Leontopodium alpinium*), white lotus (e.g. nymphaea alba), turmeric root, marshmallow (e.g. *Althaea officianlis*), burdock (e.g. *Arctium lappa*), bilberry (e.g. *Vaccinium myrtillus*), cranberry (e.g. *Vaccinium oxycoccus*), pomegranate (e.g. *Punica granatum*), sage (e.g. *Salvia officianlis*), thyme (e.g. *Thymus vulgaris*), sunflower (e.g. *Helianthus annus*), wild carrot (e.g. *Daucus carota*), hop (e.g. *Humulus lupulus*), witch hazel (e.g. *Hamamelis*), oak (e.g. *Quercus*), Camellia (e.g. *Theacea*), red clover (e.g. *Tritolium pratense*), flax (e.g. *Linium usitatissiumum*), lemon (e.g. *Citrus limon*), birch (e.g. *Betula),* cornflower (e.g. *Centaurea cyanus),* geranium, polygonum, soy (e.g. *Glycine max*), Sophora and combinations thereof. In one embodiment where one or more antioxidant agents are present in the cosmetic composition, the one or more antioxidant agents are present in an amount of about 0.1% to about 10% by weight of the composition, or about 0.1% to about 8% by weight of the composition, or about 1% to about 5% by weight of the composition. In one embodiment where one or more antioxidant agents are present in the cosmetic composition, the antioxidant agents comprise extracts of eucalyptus (e.g. *Eucalyptus globulus*), chamomile (e.g. *Anthenis nobilis*), rosemary (e.g. *Rosemarinus officialis*) and/or hop (e.g. *Humulus lupulus*).

The cosmetic composition of the invention may further comprise one or more vitamins. For example, the cosmetic composition may further comprise vitamin B, vitamin B1 to vitamin B12, vitamin C, vitamin D, vitamin E, vitamin K, vitamin H, derivatives thereof, provitamins thereof (e.g. provitamin B5 (panthenol)), or combinations thereof. In embodiments where one or more vitamins are present in the cosmetic composition, the one or more vitamins may be present in an amount of about 0.0001% to about 50% by weight of the composition, about 0.001% to about 10% by weight of the composition, about 0.01% to about 8% by weight of the composition, or about 0.1% to about 5% by weight of the composition. In one embodiment where one or more vitamins are present in the cosmetic composition, the one or more vitamins are present in an amount of about 0.1% to about 5% by weight of the composition. In one embodiment where one or more vitamins are present, the vitamin is vitamin C or a derivative thereof.

The cosmetic composition of the invention may further comprise one or more sunscreen agents, including but not limited to inorganic sunscreen agents (e.g. microfine titanium dioxide, microfine zinc oxide, iron oxides, talcs and/or boron nitride) and organic sunscreen agents (e.g. p-aminobenzoic acids, esters and derivatives thereof (e.g. 2-ethylhexyl p-dimethyl-aminobenzoate), methoxycinnamate esters (e.g., 2-ethylhexyl p-methoxycinnamate, 2-ethoxyethyl p-methoxycinnamate or α,β-di-(p-methoxycinnamoyl)-α'-(2ethylhexanoyl)-glycerin), benzophenones (e.g. oxybenzone), dibenzoylmethanes (e.g. 4-(tert-butyl)-4'-methoxydibenzoylmethane), 2-phenylbenzimidazole-5 sulfonic acid and salts thereof, alkyl-β,β-diphenylacrylates (e.g. alkyl α-cyano-β,β-diphenylacrylates such as octocrylene) triazines (such as 2,4,6-trianilino-(p-carbo-2-ethyl-hexyl-1-oxy)-1,3,5 triazine), and/or camphor derivatives (such as methylbenzylidene camphor). In embodiments where one or more sunscreen agents are present in the cosmetic composition, the one or more sunscreen agents may be present in an amount of about 0.01 to about 10% by weight of the composition.

The cosmetic composition of the invention may further comprise one or more pH adjusting agents, including but not limited to potassium hydroxide, sodium hydroxide, aminomethyl propanol, citric acid, sodium citrate, and/or triethanolamine. The cosmetic composition of the invention may have a pH from about 3 to about 10, e.g. from about 4 to about 8, or from about 5 to about 7. Preferably, the pH of the composition is about 5 to about 5.5. In embodiments where one or more pH adjusting agents are present in the cosmetic composition, the one or more pH adjusting agents may be present in an amount of from about 0.01 to about 10% by weight of the composition.

The cosmetic composition of the invention may further comprise one or more thickeners or gelling agents. In one embodiment, the thickener is selected from the group consisting of Cocamide MEA, Glyceryl Laurate, Glyceryl Oleate, Laureth-3, Laureth-4, PEG-7 Glyceryl Cocoate, PEG-18 Glyceryl Oleate, PEG-18 Glyceryl Cocoate, PEG-120 Methyl Glucose Dioleate, PEG-120 Methyl Glucose Trioleate, PEG-150 Distearate, PEG-200 Hydrogenated Glyceryl Palmate, Acrylates Copolymer, Acrylates/C10-30 Alkyl Acrylate Crosspolymer, Acrylates Crosspolymer-4, Carbomer, Hydroxyethyl Cellulose, Magnesium Aluminium Silicate, Xanthan Gum, or combinations thereof. In one embodiment, the thickener is selected from the group consisting of Cocamide MEA, Glyceryl Laurate, Glyceryl Oleate, Laureth-3, Laureth-4, PEG-7 Glyceryl Cocoate, PEG-18 Glyceryl Oleate, PEG-18 Glyceryl Cocoate, PEG-120 Methyl Glucose Dioleate, PEG-120 Methyl Glucose Trioleate, PEG-150 Distearate, PEG-200 Hydrogenated Glyceryl Palmate, or combinations thereof. In embodiments where one or more thickeners/gelling agents are present in the cosmetic composition, the one or more thickeners/gelling agents may be present in an amount of about 0.01 to about 10% by weight of the composition.

The cosmetic compositions of the invention may further comprise one or more perfumes and/or colourings.

In the present application, the term "about" may encompass ±10%, such as ±5%, e.g. ±2% or ±1%.

The skilled person will understand that optional features of one embodiment or aspect of the invention may be applicable, where appropriate, to other embodiments or aspects of the invention.

### Examples

### Test for efficacy of antimicrobial preservation³

The efficacy of antimicrobial preservation in cosmetic compositions was tested.

The test consisted of challenging a cosmetic composition (comprising a cosmetically acceptable carrier and preservative ingredients) with an inoculum of suitable microorganisms, storing the inoculated composition at a specified temperature, obtaining samples from the inoculated composition at specified time intervals and counting the microorganisms in the samples obtained.

The below method was followed.

### Method

1. Inoculate a cosmetic composition with a suspension of a test organism (i.e. *E*. *gergoviae, Pseudomonas aeruginosa, Staphylococcus aureus, Escherichia coli, Candida albicans,* or *Aspergillus brasiliensis*) to give an inoculum of 10⁵ to 10⁶ microorganisms per millilitre of the composition. The volume of the suspension of inoculum does not exceed 1 per cent of the volume of the composition.
2. Mix thoroughly to ensure homogeneous distribution.
3. Store the inoculated product at 20-25°C, away from light.
4. Obtain a ImL sample of the composition at zero hour and at time intervals thereafter, i.e. 2 days, 7 days, 14 days, and 28 days.
5. Determine the number of viable microorganisms by plate count.
6. Repeat for the other test organisms.

All cosmetic compositions in the following experiments were tested in accordance with the above method to determine the preservative effect of the ingredients contained therein. All tested compositions were made up of the same ingredients at the same levels, with the following exceptions to the preservative components.

### Experiment 1

In this experiment, the cosmetic compositions were tested against *E. gergoviae,* and each of the standard challenge test microorganisms *P. aeruginosa, S. aureus, E. coli, C. albicans,* and *A. brasiliensis.*

### Composition 1a

The first cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent potassium sorbate (PS) at a level of 0.075% by weight of the composition and also the chelating agent tetrasodium EDTA at a level of 0.05% by weight of the composition.

### Composition 1b (comparative)

The other cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent potassium sorbate (PS) at a level of 0.075% by weight of the composition and also the chelating agent tetrasodium glutamate diacetate (tetrasodium GLDA) at a level of 0.07% by weight of the composition.

### Experiment 2

In this experiment, the cosmetic compositions were tested against *E. gergoviae,* and each of the standard challenge test microorganisms *P. aeruginosa, S. aureus, E. coli, C. albicans,* and *A. brasiliensis.*

### Composition 2a

The first cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent polyaminopropyl biguanide (PHMB) at a level of 0.0075% by weight of the composition and also the chelating agent tetrasodium EDTA at a level of 0.05% by weight of the composition.

### Composition 2b (comparative)

The other cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent polyaminopropyl biguanide (PHMB) at a level of 0.015% by weight of the composition and also the chelating agent tetrasodium glutamate diacetate (tetrasodium GLDA) at a level of 0.07% by weight of the composition.

### Experiment 3

In this experiment, the cosmetic compositions were tested against *E. gergoviae.*

### Composition 3a

The first cosmetic composition tested contained a core blend of antimicrobial agents (phenoxyethanol (PE), benzoic acid (BA) and dehydroacetic acid (DHA)) at a combined level of 0.6% by weight of the composition and a blend of antimicrobial booster agents ethylhexylglycerin (EHG) and caprylyl glycol (CG) at a combined level of 0.4% by weight of the composition. The composition further contained the antimicrobial agent sodium benzoate (SB) at a level of 0.1% by weight of the composition and also the chelating agent tetrasodium EDTA at a level of 0.05% by weight of the composition.

### Composition 3b (comparative)

Another cosmetic composition tested contained a core blend of antimicrobial agents (phenoxyethanol (PE), benzoic acid (BA) and dehydroacetic acid (DHA)) at a combined level of 0.6% by weight of the composition and a blend of antimicrobial booster agents ethylhexylglycerin (EHG) and caprylyl glycol (CG) at a combined level of 0.4% by weight of the composition. The composition further contained the antimicrobial agent sodium benzoate (SB) at a level of 0.1% by weight of the composition and also the chelating agent tetrasodium glutamate diacetate (tetrasodium GLDA) at a level of 0.07% by weight of the composition.

### Composition 3c (comparative)

Another cosmetic composition tested contained a core blend of antimicrobial agents (phenoxyethanol (PE), benzoic acid (BA) and dehydroacetic acid (DHA)) at a combined level of 0.6% by weight of the composition and a blend of antimicrobial booster agents ethylhexylglycerin (EHG) and caprylyl glycol (CG) at a combined level of 0.4% by weight of the composition. The composition further contained the antimicrobial agent sodium benzoate (SB) at a level of 0.1% by weight of the composition and also the chelating agent trisodium ethylenediamine disuccinate (TEDD) at a level of 0.12% by weight of the composition.

### Composition 3d (comparative)

Another cosmetic composition tested contained a core blend of antimicrobial agents (phenoxyethanol (PE), benzoic acid (BA) and dehydroacetic acid (DHA)) at a combined level of 0.6% by weight of the composition and a blend of antimicrobial booster agents ethylhexylglycerin (EHG) and caprylyl glycol (CG) at a combined level of 0.4% by weight of the composition. The composition further contained the antimicrobial agent sodium benzoate (SB) at a level of 0.1% by weight of the composition. No chelating agent was present.

### Experiment 4

In this experiment, the cosmetic compositions were tested against *E. gergoviae* and each of the standard challenge test microorganisms *P. aeruginosa, S. aureus, E. coli, C. albicans,* and *A. brasiliensis.*

### Composition 4a

The first cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent chlorhexidine digluconate (CHG) at a level of 0.025% by weight of the composition and also the chelating agent tetrasodium EDTA at a level of 0.05% by weight of the composition.

### Composition 4b

Another cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent chlorhexidine digluconate (CHG) at a level of 0.015% by weight of the composition and also the chelating agent tetrasodium EDTA at a level of 0.05% by weight of the composition.

### Composition 4c (comparative)

Another cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and the antimicrobial booster agent ethylhexylglycerin (EHG) at a level of 0.1% by weight of the composition. The composition further contained the antimicrobial agent chlorhexidine digluconate (CHG) at a level of 0.025% by weight of the composition and also the chelating agent tetrasodium glutamate diacetate (tetrasodium GLDA) at a level of 0.07% by weight of the composition.

### Experiment 5

In this experiment, the cosmetic compositions were tested against *E. gergoviae* and each of the standard challenge test microorganisms *P. aeruginosa, S. aureus, E. coli, C. albicans,* and *A. brasiliensis.*

### Composition 5a

The first cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and a blend of antimicrobial booster agents ethylhexylglycerin (EHG) and caprylyl glycol (CG) at a combined level of 0.4% by weight of the composition. The composition further contained the antimicrobial agent chlorphensin (CPN) at a level of 0.1% by weight of the composition and also the chelating agent tetrasodium EDTA at a level of 0.05% by weight of the composition.

### Composition 5b

Another cosmetic composition tested contained a core blend of antimicrobial agents (phenoxyethanol (PE), benzoic acid (BA), dehydroacetic acid (DHA) and sodium dehydroacetate (NaDH)) at a combined level of 0.76% by weight of the composition and a blend of antimicrobial booster agents ethylhexylglycerin (EHG) and caprylyl glycol (CG) at a combined level of 0.4% by weight of the composition. The composition further contained the antimicrobial agent chlorphensin (CPN) at a level of 0.1% by weight of the composition and also the chelating agent tetrasodium glutamate diacetate (tetrasodium GLDA) at a level of 0.07% by weight of the composition.

### Acceptance criteria

The criteria for evaluation of antimicrobial activity is given in the below table in terms of the log₁₀ reduction in the number of viable microorganisms against the value obtained for the inoculum. The values represent the minimum required to achieve these criteria. The antimicrobial activity of the above tested compositions was evaluated using these criteria.

| **Product Type** | **Inoculum** | **2 day** | **7 day** | **14 day** | **28 day** |
|---|---|---|---|---|---|
| **Topical Preparations** | Bacteria | 2.0 | 3.0 | - | NI |
| | Criteria A | | | | |
| | Bacteria | - | - | 3.0 | NI |
| | Criteria B | | | | |
| | Yeasts | - | - | 2.0 | NI |
| | Moulds | | | | |
| | Criteria A | | | | |
| | Yeasts | - | - | 1.0 | NI |
| | Moulds | | | | |
| | Criteria B | | | | |

The A criteria is the recommended efficacy to be achieved.

In justified cases where the A criteria cannot be achieved, e.g. for reasons of an increased risk of adverse reactions, the B criteria must be satisfied.

NI: no increase in the number of viable microorganisms compared to the previous reading.

### Results

### Experiments 1 and 2

Composition 1a, which contained an EDTA derivative, achieved an A criteria pass for *E*. *gergoviae* and all standard challenge test microorganisms. Composition 1b, which lacked an EDTA derivative, failed the antimicrobial efficacy test for *E. gergoviae* and all standard challenge test microorganisms.

With reference to Experiment 2, Composition 2a, which contained an EDTA derivative, achieved an A criteria pass for *E. gergoviae* and all standard challenge test microorganisms. Composition 2b, which lacked an EDTA derivative, achieved an A criteria pass for *E. gergoviae* but failed on the standard challenge test for *A.brasiliensis* and *E.coli.*

The absence of an EDTA derivative in Compositions 1b and 2b was detrimental to preservative effect. However, unexpectedly, when an EDTA derivative was used, acceptable microbial loads were achieved for *E. gergoviae* and all standard challenge test microorganisms (see Compositions 1a and 2a). Even more surprisingly was the finding that the overall level of antimicrobial agent can be reduced, in fact halved, when an EDTA derivative is used (0.0075% PHMB in Composition 2a vs. 0.015% PHMB in Composition 2b), yet the A criteria pass rating can still be achieved for all test organisms.

### Experiment 3

Composition 3a, which contained an EDTA derivative, achieved an A criteria pass for *E. gergoviae.* Compositions 3b-3d, which each lacked an EDTA derivative, all failed the antimicrobial efficacy test for *E. gergoviae.*

Again, the absence of an EDTA derivative in Compositions 3b-3d was detrimental to preservative effect. However, unexpectedly, when an EDTA derivative was used, acceptable microbial loads were achieved for *E. gergoviae.* Even a higher overall antimicrobial agent level in Composition 3d was not sufficient to achieve the pass criteria in the absence of an EDTA derivative.

### Experiment 4

Compositions 4a and 4b, both of which contained an EDTA derivative, achieved an A criteria pass for *E. gergoviae* and all standard challenge test microorganisms. Composition 4c, which lacked an EDTA derivative, failed the antimicrobial efficacy test for *E. gergoviae* and all standard challenge test microorganisms.

Despite the higher overall level of antimicrobial agent present in Composition 4c, it was not able to reduce/maintain the amount of *E. gergoviae* and the standard challenge test microorganisms to below the cosmetically acceptable level. The absence of an EDTA derivative in Composition 4c was detrimental to its preservative effect. However, unexpectedly, when an EDTA derivative was used as the chelating agent in Compositions 4a and 4b, acceptable microbial loads were achieved for *E. gergoviae* and all standard challenge test microorganisms. Even more surprisingly was the finding that the overall level of antimicrobial agent can be reduced (0.025% CHG in Composition 4a vs. 0.015% CHG in Composition 4b), yet the A criteria pass rating can still be achieved for all test microorganisms.

### Experiment 5

Composition 5a, which contained an EDTA derivative, achieved an A criteria pass for *E. gergoviae* and all standard challenge test microorganisms. Composition 5b, which lacked an EDTA derivative, failed the antimicrobial efficacy test for *E. gergoviae* and all standard challenge test microorganisms.

The absence of an EDTA derivative in Composition 5b was detrimental to preservative effect. However, unexpectedly, when an EDTA derivative was used in Composition 5a, acceptable microbial loads were achieved for *E. gergoviae* and all standard challenge test microorganisms.

The below represent non-binding examples of cosmetic compositions of the invention.

**Formulation Example 1 - Skin Cream**

| **Material** | **% w/w** |
|---|---|
| Caprylic/capric triglyceride | 10 |
| Cetearyl alcohol | 2 |
| Ethylhexylglycerin | 0.15 |
| Glyceryl stearate | 2 |
| Cetyl alcohol | 2 |
| PEG-100 stearate | 2 |
| Dimethicone | 1.5 |
| PEG-20 stearate | 0.5 |
| Phenoxyethanol | 0.4 |
| Carbomer | 0.2 |
| Methylparaben | 0.2 |
| Ethylparaben | 0.15 |
| Potassium hydroxide | 0.06 |
| Potassium hydroxide | 0.015 |
| Alcohol denat. | 0.5 |
| Tetrasodium EDTA | 0.05 |
| Chlorhexidine digluconate | 0.075 |
| Ascorbyl glucoside | 0.05 |
| Gingko extract | 0.005 |
| Emblica extract | 0.015 |
| Dimethylmethoxy chromanol | 0.02 |
| White lupin peptides | 1 |
| Palmitoyl oligopeptide and Palmitoyl tetrapeptides | 1.5 |
| Retinyl palmitate | 0.07 |
| Aqua | To 100 |

### Method of manufacture:

1. To water add and dissolve tetrasodium EDTA.
2. Using homogenisation sprinkle in carbomer and continue to homogenise for 5 minutes or until hydrated.
3. Add methylparaben and ethylparaben and heat up to 70-75°C.
4. In a separate vessel weigh out oil phase and heat to 70-75°C. (Caprylic/capric triglyceride, cetearyl alcohol, glyceryl stearate, cetyl alcohol, PEG-100 stearate, dimethicone, PEG-20 stearate and retinyl palmitate)
5. With both phases at 70-75°C add the oil phase to the water phase and homogenise for 2 minutes.
6. Add potassium hydroxide and homogenise for 2 minutes.
7. Cool to room temperature.
8. Stir in phenoxyethanol and Chlorhexidine digluconate.
9. Dissolve ascorbyl glucoside in 2% water, neutralise with potassium hydroxide and stir into bulk.
10. Dissolve gingko extract in ethylhexylglycerin and stir into bulk.
11. Dissolve emblica extract in 2% water and stir into bulk.
12. Dissolve dimethylmethoxy chromanol in alcohol denat. and stir into bulk.
13. Stir into bulk white lupin peptides, palmitoyl oligopeptide and palmitoyl tetrapeptides.
14. Make to weight with water and stir smooth.

**Formulation Example 2 - Skin Cream with SPF**

| **Material** | **% w/w** |
|---|---|
| C12-15 alkyl benzoate | 5 |
| Butyl methoxydibenzoylmethane | 3 |
| Ethylhexyl methoxycinnamate | 5 |
| Cetearyl alcohol | 2 |
| Ethylhexylglycerin | 0.15 |
| Glyceryl stearate | 2 |
| Cetyl alcohol | 2 |
| PEG-100 stearate | 2 |
| Dimethicone | 1.5 |
| PEG-20 stearate | 0.5 |
| Phenoxyethanol | 0.4 |
| Carbomer | 0.2 |
| Methylparaben | 0.2 |
| Ethylparaben | 0.15 |
| Potassium hydroxide | 0.06 |
| Alcohol denat. | 0.5 |
| Chlorhexidine digluconate | 0.075 |
| Tetrasodium EDTA | 0.05 |
| Aqua | To 100 |

**Formulation Example 3 - Gel**

| **Material** | **% w/w** |
|---|---|
| Ethylhexylglycerin | 0.2 |
| Acrylates/C10-30 alkyl acrylate crosspolymer | 1 |
| Alcohol denat. | 0.5 |
| Phenoxyethanol | 0.4 |
| Potassium hydroxide | 0.29 |
| Tetrasodium EDTA | 0.05 |
| Chlorhexidine digluconate | 0.075 |
| Methylparaben | 0.08 |
| Ethylparaben | 0.05 |
| Aqua | To 100 |

**Formulation Example 4 - Hair Conditioner**

| **Material** | **% w/w** |
|---|---|
| Cetyl alcohol | 4 |
| Cetrimonium chloride | 3 |
| Phenoxyethanol | 0.6 |
| Propanediol | 1 |
| Tetrasodium EDTA | 0.05 |
| Chlorhexidine digluconate | 0.075 |
| Aqua | To 100 |

**Formulation Example 5 - Body Wash**

| **Material** | **% w/w** |
|---|---|
| Sodium laureth sulphate | 36 |
| Cocamidopropyl betaine | 4 |
| Cocamide DEA | 1.5 |
| Sodium chloride | 1.5 |
| Sodium benzoate | 0.3 |
| Sodium methylparaben | 0.2 |
| Tetrasodium EDTA | 0.05 |
| Citric acid | 0.05 |
| Sodium hydroxide | 0.05 |
| Hydroxy acetophenone | 0.2 |
| Chlorhexidine digluconate | 0.075 |
| Aqua | To 100 |

**Formulation Example 6 - Water-in-silicone Foundation Emulsion with SPF**

| **Material** | **% w/w** |
|---|---|
| Aqua | To 100 |
| Cyclopentasiloxane | 20 |
| Ethylhexyl methoxycinnamate | 5 |
| Talc | 4 |
| Ethylhexyl stearate | 3 |
| Mica | 3 |
| Cyclohexasiloxane | 3 |
| Dimethicone crosspolymer | 3 |
| Dimethicone | 2.5 |
| Silica | 2 |
| Titanium dioxide | 2 |
| Butylene glycol | 2 |
| Disteardimonium hectorite | 1.5 |
| Dimethicone copolyol | 1.2 |
| Magnesium sulfate | 1 |
| Phenoxyethanol | 0.6 |
| Cetyl PEG/PPG-10/1 dimethicone | 0.5 |
| Hexyl Laurate | 0.5 |
| Polyglyceryl-4 isostearte | 0.5 |
| Propylene carbonate | 0.3 |
| Stearic acid | 0.15 |
| Triethoxycaprylylsilane | 0.13 |
| p-anisic acid | 0.1 |
| Methyl paraben | 0.13 |
| Ethyl paraben | 0.03 |
| Chlorhexidine digluconate | 0.075 |
| Tetrasodium EDTA | 0.02 |
| Pigment | 10 |

**Formulation Example 7 - Eye Cream**

| **Material** | **% w/w** |
|---|---|
| Aqua | To 100 |
| Caprylic/capric triglyceride | 5 |
| Ethylhexylglycerin | 0.1 |
| Butyrospermum parkii (shea) butter | 3 |
| Helianthus annuus seed oil | 1 |
| Cetearyl alcohol | 2.4 |
| Paraffin | 2 |
| Dimethicone | 1 |
| Glyceryl stearate | 1 |
| Cetyl alcohol | 1 |
| Phenoxyethanol | 0.6 |
| PEG-20 stearate | 0.6 |
| Methylparaben | 0.25 |
| Ethylparaben | 0.1 |
| Carbomer | 0.2 |
| Potassium hydroxide | 0.07 |
| Chlorhexidine digluconate | 0.075 |
| Tetrasodium EDTA | 0.05 |

**Formulation Example 8 - Toner**

| **Material** | **% w/w** |
|---|---|
| Aqua | To 100 |
| Sodium citrate | 0.1 |
| Citric acid | 0.1 |
| Ethylhexylglycerin/Caprylyl glycol | 0.4 |
| Polysorbate 20 | 0.5 |
| PEG-40 hydrogenated castor oil | 1 |
| Chlorhexidine digluconate | 0.075 |
| Tetrasodium EDTA | 0.05 |
| Tocopheryl acetate | 0.1 |
| Fragrance | 0.1 |
| Sodium benzoate | 0.2 |
| Alcohol denat. | 5 |
| Phenoxyethanol | 0.5 |

**Formulation Example 9 - Cleansing water**

| **Material** | **% w/w** |
|---|---|
| Aqua | To 100 |
| Potassium hydroxide | 0.07 |
| Citric acid | 0.1 |
| Caprylhydroxamic acid | 0.1 |
| PEG-6 caprylic/capric glycerides | 2 |
| PEG-40 hydrogenated castor oil | 1 |
| Chlorhexidine digluconate | 0.075 |
| Tetrasodium EDTA | 0.05 |
| Tocopheryl acetate | 0.1 |
| Fragrance | 0.1 |
| Sodium benzoate | 0.2 |
| Phenoxyethanol | 0.5 |

### References

1. Orus, P. et al. Increasing antibiotic resistance in preservative-tolerant bacterial strains isolated from cosmetic products. International Micobiology, 18, 51-59 (2015).
2. Périamé, M. et al. Enterobacter gergoviae adaptation to preservatives commonly used in cosmetic industry. International Journal of Cosmetic Science 36, 386-395 (2014).
3. European Pharmacopoeia 8.0, Section 5.1.3

## Claims

1. A cosmetic composition comprising a cosmetically acceptable carrier and a preservative system, wherein the preservative system comprises:
(i) an antimicrobial agent;
(ii) a chelating agent comprising ethylenediamine tetraacetic acid (EDTA) or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% by weight of the composition.

2. The cosmetic composition of claim 1, wherein the chelating agent ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, or combinations thereof.

3. The cosmetic composition of claim 2, wherein the chelating agent is selected from the group consisting of disodium EDTA, trisodium EDTA, tetrasodium EDTA, and combinations thereof.

4. The cosmetic composition of any one of the preceding claims, wherein the antimicrobial agent comprises chlorhexidine or a salt thereof, chlorphensin, benzoic acid or a salt or ester thereof, sorbic acid or a salt thereof, 4-hydroxybenzoic acid or a salt or ester thereof, dehydroacetic acid or a salt thereof, bronopol, benzyl alcohol, polyaminopropyl biguanide, phenoxyethanol, or combinations thereof.

5. The cosmetic composition of claim 4, wherein the antimicrobial agent comprises phenoxyethanol, benzoic acid, dehydroacetic acid, potassium sorbate, sodium dehydroacetate, sodium benzoate, chlorphensin, chlorhexidine digluconate, and/or polyaminopropyl biguanide.

6. The cosmetic composition of claim 5, wherein the antimicrobial agent comprises potassium sorbate and/or polyaminopropyl biguanide.

7. The cosmetic composition of any one of the preceding claims, wherein the antimicrobial agent is present in an amount of:
(a) from about 0.01% to about 1% by weight of the composition;
(b) from about 0.1% to about 1% by weight of the composition; or
(c) from about 0.3% to about 0.9% by weight of the composition.

8. The cosmetic composition of any one of the preceding claims, wherein the chelating agent is present in an amount of:
(a) from about 0.001% to about 5% by weight of the composition;
(b) from about 0.005% to about 1% by weight of the composition; or
(c) from about 0.01% to about 0.3% by weight of the composition.

9. The cosmetic composition of any one of the preceding claims, wherein the antimicrobial booster agent comprises ethylhexylglycerin, propanediol and/or caprylyl glycol.

10. The cosmetic composition of any one of the preceding claims, wherein the antimicrobial booster agent is present in an amount of:
(a) from about 0.001% to about 5% by weight of the composition;
(b) from about 0.005% to about 1% by weight of the composition; or
(c) from about 0.01% to about 0.8% by weight of the composition.

11. The cosmetic composition of any one of claims 8 to 10, wherein:
(i) the antimicrobial agent comprises phenoxyethanol, benzoic acid, dehydroacetic acid, potassium sorbate, sodium dehydroacetate, sodium benzoate, chlorphensin, chlorhexidine digluconate, and/or polyaminopropyl biguanide;
(ii) the chelating agent comprises tetrasodium EDTA; and
(iii) the antimicrobial booster agent comprises ethylhexylglycerin and/or caprylyl glycol.

12. The cosmetic composition of claim 11, wherein the total amount of antimicrobial agent is present at a level of from about 0.01% to about 0.9% by weight of the total weight of the composition.

13. The cosmetic composition of any one of claims 8 to 10, wherein:
(i) the antimicrobial agent is selected from the group consisting of chlorhexidine or a salt thereof, chlorphensin, benzoic acid and/or a salt or ester thereof, sorbic acid or a salt thereof, 4-hydroxybenzoic acid or a salt or ester thereof, dehydroacetic acid and/or a salt thereof, bronopol, benzyl alcohol, polyaminopropyl biguanide, phenoxyethanol, and combinations thereof; and
(ii) the chelating agent is selected from the group consisting of ethylenediamine tetraacetic acid (EDTA), dipotassium EDTA, disodium EDTA, tetrasodium EDTA, diammonium EDTA, calcium disodium EDTA, TEA-EDTA, tripotassium EDTA, trisodium EDTA, hydroxyethyl ethylenediamine triacetic acid (HEDTA), trisodium HEDTA, and combinations thereof,
wherein the only antimicrobial agent present in the cosmetic composition is that which is present in the preservative system.

14. Use of a preservative system to prevent or reduce *Enterobacter gergoviae* growth or proliferation in a cosmetic composition, wherein the preservative system comprises:
(i) an antimicrobial agent;
(ii) a chelating agent comprising ethylenediamine tetraacetic acid (EDTA) or a derivative thereof; and
(iii) an antimicrobial booster agent selected from the group consisting of ethylhexylglycerin, caprylyl glycol, hydroxy acetophenone, caprylhydroxamic acid, phenethyl alcohol, p-anisic acid, glyceryl caprylate, levulinic acid, sodium levulinate, propanediol, hexanediol, pentylene glycol, capryloyl glycine, methylpropanediol, phenylpropanol, sodium anisate, and combinations thereof,
wherein the antimicrobial agent is present in an amount of from about 0.001% to about 1% by weight of the composition.

15. The use according to claim 14, wherein the antimicrobial agent is selected from the group consisting of chlorhexidine or a salt thereof, chlorphensin, benzoic acid or a salt or ester thereof, sorbic acid or a salt thereof, 4-hydroxybenzoic acid or a salt or ester thereof, dehydroacetic acid or a salt thereof, bronopol, benzyl alcohol, polyaminopropyl biguanide, phenoxyethanol, and combinations thereof, and
optionally wherein the chelating agent is tetrasodium EDTA, and
optionally wherein the antimicrobial booster agent is selected from the group consisting of ethylhexylglycerin, caprylyl glycol, propanediol hydroxy acetophenone, caprylhydroxamic acid, phenethyl alcohol, p-anisic acid, glyceryl caprylate, and combinations thereof.
